# EUROPEAN PATENT APPLICATION

(11) **EP 1 902 711 A1**
(43) Date of publication of application: **26.03.2008**
(21) Application number: 06019635.9
(22) Date of filing: 20.09.2006
(51) Int. Cl.: A61K 31/352, A61K 31/585, A61K 9/00, A61K 9/20

(54) **Oral modified release formulations containing Drospirenon and 8-Prenylnaringenin for use in Hormone Replacement Therapy (HRT)**

(71) Applicant: KAIROSmed GmbH, 10117 Berlin (DE)
(72) Inventor: Hümpel, Michael, Dr., 14163 Berlin (DE); Schleuning, Wolf-Dieter, Dr., 13467 Berlin (DE); Heuermann, Arno, Dipl.-Ing., 10115 Berlin (DE); Krings, Matthias, Dr., 80992 München (DE); Thunecke, Markus, Dr., 14193 Berlin (DE)
(74) Representative: Jungblut & Seuss

(57) **Abstract**

This invention is directed to an oral modified release formulation of the phytoestrogen 8-Prenylnaringenin in combination with a progestin, preferably with Drospirenone, and its use for combination hormone replacement therapy.

## Description

This invention relates to oral modified release formulations containing combinations of progestins, and preferably the combination of the synthetic progestin Drospirenon (DRSP), and the estrogenic natural compound 8-Prenylnaringenin (8-PN) and their use in hormone replacement therapy (HRT) of menopausal women.

Estrogen deficiency in females may have different causes, e.g. inactive or surgically removed ovaries or the cease of estrogen production in the menopause. One medical intervention to treat the negative effects of estrogen deficiency consists of replacing the missing estrogens (estradiol (E2), estrone (E1), estriol (E3)) or to use synthetic estrogens like ethinyl estradiol or natural conjugated (equine) estrogens (CEE). The main objective of these compounds is the replacement of estrogen activity in the menopause (Hormone Replacement Therapy, HRT). All these compounds are effective as treatments of menopausal symptoms (short term) and the treatment and prevention of osteoporosis (short and long term). Recently, a new estrogenic compound, 8-Prenylnaringenin, discovered in plants (hops *(Humulus lupulus),* and a traditional Siamese medicinal plant *(Anaxagorea luzonensis* A. Gray)) was shown to be not only the most active phyto-estrogen known so far but also a substance exhibiting tissue specificity with a much lower activity on uterine growth and endometrial stimulation than estradiol at equivalent bone protective doses. On the basis of this new pharmacological profile it was claimed that relief of menopausal symptoms and the treatment and protection of osteoporosis are possible without the concomitant administration of a progestin and therefore without the unwanted withdrawal bleedings (WO 2005/037816). Additionally, the unique pharmacokinetic properties of 8-PN detected in women led to the invention of an oral modified release formulation containing 8-Prenylnaringenin and its use for the treatment of symptoms of estrogen deficiency (EP 05090049.7).

Generally spoken, there are two main medical strategies to manage the up to 35 menopausal symptoms (AWARE™).
The first strategy is to replace the missing estrogen by any route of administration and using various pharmaceutical products. Examples are Estrace® or Estradiol generic® (E2 oral, E2 in vaginal cream), Premarin® (CEE oral, CEE in vaginal cream), Estratab®, Menest®, Ogen®, Ortho-Est® (esterified estrogens (mostly E1) oral), Tri-Est®, Bi-Est® (mixtures of E1 and E2 or E1, E2 and E3 oral), and Climara®, Alora®, Estraderm®, FemPatch®, Vivelle® (E2 as transdermal delivery system). Whereas these products are effective in the management of menopausal symptoms they constitute a health risk for women maintaining an intact uterus (promotion and development of endometrial cancer). Hence, an E1, E2, E3 or CEE based mono-hormonal HRT is indicated for women after hysterectomy or requires occasional combination with a progestin. Menostar® is a recent TDS product only releasing 15µg E2/d and it is claimed that this small dose is sufficient to protect against hormone dependent osteoporosis without stimulation of the endometrium.
The alternative strategy is the combination of estrogen with a progestin in order to avoid endometrial stimulation by unopposed estrogen. Examples for oral preparations are PremPro® (CEE plus MPA), Ortho-Prefest® (E2 plus norgestimate), Kliogest® (E2 plus Norethisterone), Nuvelle® (E2-ester plus levonorgestrel), Climen® (E2-ester plus cyproterone acetate), Angeliq (E2 plus Drospirenone), Climodien (E2-ester plus Dienogest). In addition, transdermal application systems releasing a progestin and estradiol are available (CombiPatch® E2 plus NetAc). Combination products are either used in a cyclical mode (7 days drug-free interval) or continuously.
Combination HRT preparations have been known for decades and the numerous preparations currently in use mainly differ in the type of progestin. A continuous treatment schedule (without a drug-free interval of normally 7 days) that avoids bleeding periods has been developed recently. This is achieved by the preponderance of the progestin rendering the endometrium atrophic after several months of use. Therefore, vaginal bleedings diminish over time and stop almost completely after 6 to 12 months. The only necessary pharmacological effect of the progestin in combination HRT products is to counteract the estrogen at the endometrial level. Therefore the daily dose of any of the mentioned progestins in HRT products is about 30 % lower than their respective dose in combination oral contraceptives. A tissue specific estrogen such as 8-PN in a combination HRT preparation may require substantially less progestin because of the marginal or absent stimulatory effect on the endometrium.
Looking at the known progestins there are some differences in pharmacological profiles. The 19-nor-testosterone derivative Norethisterone (NET), its pro-drugs NET-AC, lynestrenol, and norethinodrel or other derivatives of the same chemical class (e.g. desogestrel, norgestimate, norgestrel, levonorgestrel) and their active metabolites are characterized by their partial androgenic activity leading to numerous unwanted effects among which sebaceous skin and body weight gain are the most dreaded ones. Other progestins are derivatives of progesterone (the so-called C-21 progestins) and medroxyprogesterone acetate (MPA), chlormadinone acetate (CMA) and cyproterone acetate (CPA) are the best known representatives of this chemical class. Like the natural progesterone these compounds do not activate the androgen receptor and consequently do not act as androgen. In contrast, MPA, CMA and - more pronounced - CPA show a partial anti-androgenic activity which can be used for the treatment of acne and other symptoms generated by enhanced testosterone levels in women. However, all these progestins lack the anti-mineral-corticoid activity of progesterone. Just recently such a progestin was found as a progestagenic metabolite of a spironolactone derivative. Drospirenone (DRSP) has been characterized as a highly potent progestin with some anti-androgenic and aldosterone antagonistic activities and thus almost perfectly mimicks the pharmacological profile of natural progesterone. The structure of DRSP is as follows:

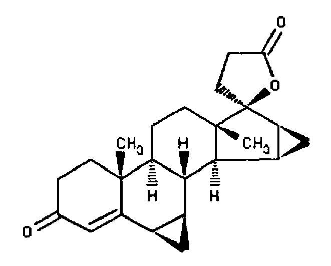

The aldosterone-antagonistic activity of DRSP were the subject of a patent application in 1976 (Schering AG). Another patent application was filed in 1977 claiming the substance as a diuretic drug. Its use as a progestin in oral contraception and gynecological indications was patented in 1980. Thus, the patent protection of the substance ran out at the same time when the drug came to the market as constituent of the combined oral contraceptive Yasmin® (introduction into the market in Germany was November 2000). The patent protection of Yasmin® is mainly based on a technical patent claiming a stable oral formulation with acute drug release (US 6,787,531).
DRSP 2 mg in combination with 1.0 mg E2 is on the market as Angeliq® since 2005. Treatment is continuous. After 6 or 12 months of use no bleedings and an atrophic endometrium are found in 85 or 90 % of women. Main menopausal symptoms like hot flashes, sleep disturbances and sweating were reduced in more than 60 % of women after 1 - 4 months.
Therefore, the preferred progestin to combine with 8-PN in a HRT preparation is drospirenone (DRSP).
Besides the tissue specificity of estrogenic effects of 8-PN there is another reason for substituting E2 (or CEE, E1, E3) in oral HRT products: these compounds are clearly unsuited for oral administration. Because of the high and variable metabolism during the first liver passage estradiol exhibits extremely large variations in oral bioavailability among individuals. The coefficient of variation (CV) of the mean area under the plasma level time curve (AUC, N=14) after oral 2, 4, or 8 mg E2 accounted for 127 %, 91 %, or 70 %, respectively (Kuhnz, 1993). Compared to DRSP (CV of AUC's of about 20 %) figures show that the systemic availability of the estrogenic component in Angeliq varies much more than the progestogenic component.

These data clearly demonstrate the medical need for a substitution of E2 as the estrogen in combination oral HRT preparations. The substitute should be reliably (CV of AUC < 50 %) bioavailable after oral administration and be present at sufficiently high concentrations at the estrogen receptors (ERs) for the total treatment interval. As demonstrated below 8-PN constitutes such a suitable substitute.
Interestingly, HRT preparations are always preparations with acute drug release. This is surprising because it is well known that the dose of drugs with high oral bioavailability can be reduced by modified release administration. As an example, the oral dose of levonorgestrel as so-called minipill is 0.03 mg/d (continuous treatment; Pearl Index = 1). An even increased clinical effect (Pearl Index = 0.05-0.1) is achieved with 0.02 mg/d released from an intrauterine device (Mirena®). A non-hormonal IUD has a Pearl Index of about 2. Thus, a progestin dose sparing effect of about 30 % can be realized by modified release formulations. However, the most frequently used estrogen component in HRT products is E2 which is not a suitable drug for such a formulation because the first liver pass metabolism would degrade E2 to an even higher extent than after acute release. In consequence, to reach the same biological effect higher E2 doses would become necessary in modified release formulations and the degree of variability of systemic E2 availability would not decrease.
Recently, a new estrogen, 8-Prenylnaringenin (8-PN), found in plants (hop, *Anaxagorea luzonensis* A. Gray) was shown to be not only the most active phytoestrogen but also a substance that exhibits tissue specificity with a much lower activity on the uterus than estradiol at equivalent bone protective doses. 8-PN (5,7-Dihydroxy-2-(4-hydroxyphenyl)-8-(3-methylbut-2-enyl)-chroman-4-on) has the structure given below.
The 4-hydroxyphenyl group may either be in the 2S(-) or the 2R (+) position. 8-PN was characterized as a pure estrogen devoid of binding or activation of the PR, AR, or GR and without any anti-estogenic activity.

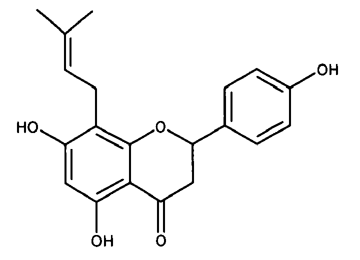

Interestingly, 8-PN is mainly inactivated by conjugation. Conjugates are excreted mainly with bile, deconjugated in the duodenum and intestinum after secretion with bile and unchanged drug is then re-absorbed. This process is known as entero-hepatic re-circulation (EHC) and is the basis for a multiple reuse of the drug in question. 8-PN shows a stable systemic availability with a CV of 42 or 29 % of mean AUC at oral doses of 250 or 750 mg, respectively. In addition, due to a surprisingly low extent of metabolism by CYP isoenzymes 8-PN shows a stable systemic availability irrespective of the mode of administration, i.e. acute or modified release. Therefore, 8-PN is a suitable drug for use in modified release formulations and can overcome the development hurdles that other combination HRT products have been facing as modified release formulations.
The normal route of administration in combination hormone replacement therapy is the oral one. This route is not only preferred because of user convenience but also because most of the substances in question need a high daily dose which is difficult to administer by alternate routes like the nasal, dermal or inhalatory ones. For the most potent natural estrogen, E2, dermal administration was shown to be an effective alternative to oral delivery because only small quantities (25 - 100 µg/d) need to reach systemic circulation. There are two reasons why the effective daily oral dose (1 or 2 mg) can be reduced by a factor of 10 - 40 when estradiol is absorbed via the skin. Most importantly, the liver, which inactivates roughly 90 % of the oral dose before reaching the systemic circulation, is bypassed by the dermal route. Thus, avoidance of the high first-liver-pass metabolism by dermal administration allows a drastic reduction of the oral dose. In addition, the continuous and steady influx of drug (zero order kinetics) over the time a patch is used (2 - 7 days) has a dose sparing effect as compared to the oral route, which is typically characterised by a superposition of several first order kinetics, i.e. absorption, distribution and disposition processes starting at a definite time of the day. This leads to steep drug serum level increases shortly after intake and subsequent decreases governed by distribution and metabolism/excretion. Because all marketed estrogens, natural or synthetic, undergo an almost complete metabolisation before excretion and show high intrinsic clearance rates, drug serum levels decrease rapidly and generally drop to very low and sometimes undetectable levels within the treatment interval of 1 day. As a result, serum (and subsequently tissue) concentrations show high fluctuations with periods of maximum effects and below threshold effects. At the doses used for estrogens on the market it can be assumed that serum and tissue concentrations fell below a maximum effective level for several hours a day. It is assumed that an effective dose can be lowered by 30 - 50 % when the total dose is fairly distributed over the total treatment interval.

A potential solution to this problem could be the use of an oral modified release formulation. These pharmaceutical formulations were developed for other drugs to avoid high plasma level fluctuations of the active compound.

The problem, therefore, is to provide an oral formulation containing 8-PN and a suitable progestin, preferably DRSP, that continuously distributes the drugs for the gastro-intestinal transit time of generally 12 - 16 hours and which preferably has to be administered once a day, preferably before bed time.
This problem was solved by a solid oral modified release formulation containing 8-PN and DRSP. This formulation contains a polymeric matrix, a buffer substance and one or more excipients in addition to 8-PN and DRSP. Preferably, the buffer substance is an alkaline substance like e. g. magnesium oxide, magnesium hydroxide, dihydroxyaluminum aminoacetate, magnesium carbonate, calcium carbonate, sodium ascorbate, magnesium trisilicate, dihydroxyaluminum sodium carbonate, aluminum hydroxide, sodium citrate, potassium phosphate, sodium bicarbonate, disodium hydrogenphosphate or some combinations thereof. Preferably, the particle size of the compounds is in the range of 0.1 - 750 µm. Most preferably, it is in the range of 20 - 400 µm. The polymer matrix is preferably chosen from the group consisting of: cellulose derivatives, acrylic derivatives, vinyl polymers, polyacrylates, polycarbonates, polyethers, polystyrenes polyanhydrides, polyesters, polyorthoesters, polysaccharides and natural polymers. Most preferably, the polymer matrix consists of water soluble polyvinylpyrrolidone and/or water insoluble polyvinylacetate. In a preferred embodiment the oral modified release formulation is coated with a polymeric coat.

Excipients may be chosen from the group consisting of lactose, calcium phosphate, manitol and starch. Preferably, an additional excipient is microcrystalline cellulose.

The solid oral modified release formulations of this invention show a pH-independent drug release in vitro. This is important as the pH varies considerably in the gastro-intestinal tract and continuous release should be achieved independent of the pH. In contrast to C21-progestins (MPA, CPA, DRSP), however, the solubility of 8-PN is pH-dependent. The compound shows higher solubility at higher pH-values whereas the solubility of the compound is low at lower pH-values. The low acid solubility property of 8-Prenylnaringenin results in vitro in slow drug dissolution at pH 1, whereas the dissolution is fast at higher pH-values such as pH 6.8. The resulting dissolution profiles are different at different pH-values. This problem is solved by the solid oral modified release formulation of this invention. In addition it is known that DRSP is sensitive to low pH-values at which the carbolactone ring can be opened to the inactive acid. It was also shown that ring opening does not occur at the pH of the stomach (no loss in bioavailability) but low pH-values have to be avoided during the manufacturing process. This problem is solved by the solid oral modified release formulation of this invention.

The solid oral modified release formulation of this invention solves the problem of a continuous distribution of 8-PN almost over 24 h. This is a combined effect of the pharmacokinetic profile of 8-Prenylnaringenin (high metabolic stability versus CYP isoenzymes, EHC) which is drastically different from those of other estrogens and the solid oral modified release formulation. The solid oral modified release formulation of this invention also solves the problem of a dose sparing effect of C21-progestins, preferably DRSP, by continuous distribution of the drug for 12 - 16 hours and increased drug trough levels at steady state conditions.

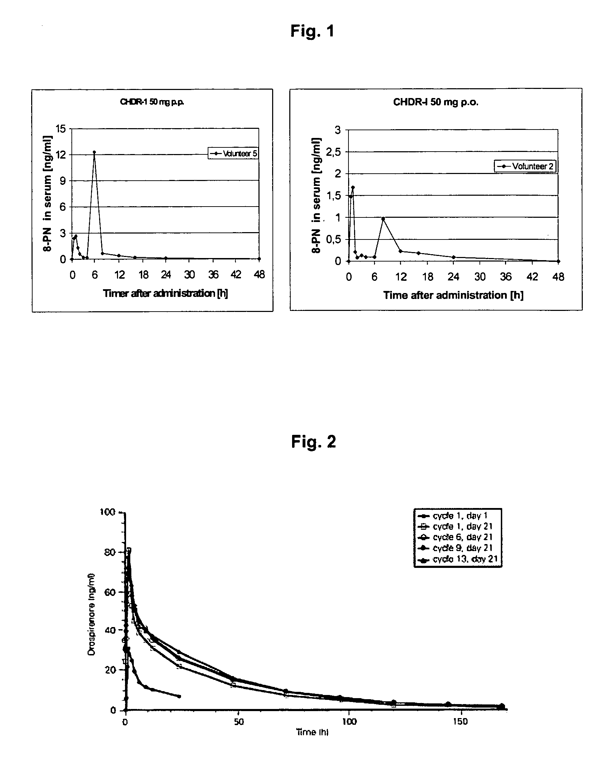

The pharmacokinetic profile of 8-PN is characterized by a complete oral absorption, a low degree of metabolisation (less than 60 % of dose) and a high pre-systemic elimination (first-liver-pass excretion; about 55 % of dose). The high and unexpected pre-systemic elimination leads to a collection of substantial dose parts in the bile fluid from which these dose parts are secreted into the duodenum when gastric signaling occurs with a meal uptake. Respective dose parts are then absorbed again and reach systemic circulation. Examples for this effect (drug serum levels) are shown in Fig. 1. Data were taken from the clinical Phase la study and represent two volunteers of the lowest dose group (50 mg 8-Prenylnaringenin).

Taking the total area under the curve as a measure for 8-Prenylnaringenin systemic availability, the percentage of the area under the second peak (generated by reabsorption of the pre-systemically eliminated and then biliary secreted dose parts) can be used to estimate those dose parts which undergo pre-systemic elimination after oral dosing. On an average of six women investigated this figure is 54 ± 20 %.

This invention is to the combination of the unexpected pharmacokinetic profile of 8-Prenylnaringenin with a modified release formulation, which enables release of the drug at an almost constant rate for 8 - 10 hours. The drug release from the solid oral modified release formulation is according to this invention preferably close to or perfect zero order kinetics. This oral modified release formulation is preferably taken in the evening (before bed-time). During nighttime 8-PN is released at almost constant rate leading to flat drug serum levels and a collection of substantial dose parts in the bile fluid which - after reabsorption - account for more than half of total systemically available dose. The next day these dose parts are secreted into the duodenum when the first meal (breakfast or lunch) is taken. Re-absorption gives rise to elevated 8-Prenylnaringenin serum levels over the first half of the day, which - at a lower level - repeats with dinner in the evening. Overall, the total systemically available dose is distributed over 24 hours avoiding unnecessary high peaks and ensuring effective drug concentrations in target tissues. The anticipated daily dose is between 25 and 250 mg, preferably between 50 and 150 mg. The oral modified release formulation is preferably taken 6 -12 hours, more preferably 8 - 12 hours before having a meal.

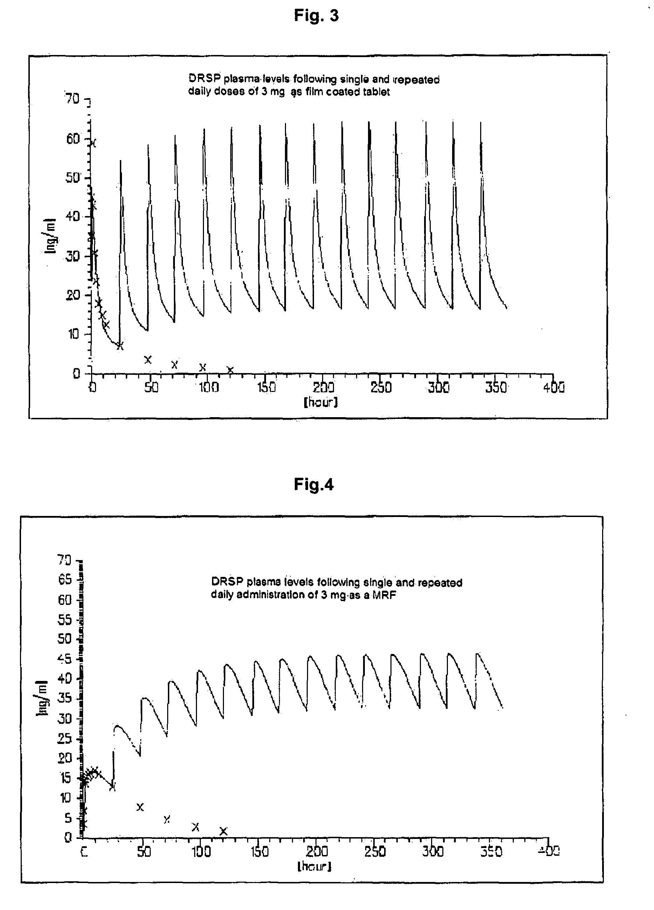

The pharmacokinetic profile of DRSP is characterized by complete oral absorption, an absolute oral bioavailability of 76 %, and a disposition half-life of 31 hours. The apparent volume of distribution was estimated to 4 L/kg. After oral intake of 3 mg DRSP maximum serum levels of 37 ng/ml were reached at 1.7 hours. AUC₀₋₂₄ₕ was 288 ngxh/ml. Following daily repeated administration the maximum serum level increased to about 80 ng/ml and AUC to about 920 ngxh/ml. Increases are the result of drug accumulation at daily treatment and a half-life of 31 hours. Figure 2 shows time course and degree of accumulation.
DRSP does not bind to SHBG and does not suppress EE induced SHBG or CBG serum levels. DRSP is only slightly metabolized, mainly by CYP3A4.
DRSP shows a 2 to 3 fold accumulation after acute drug release from the film-coated tablet. The accumulation factor (AF) of the simulated drug serum levels is 2.4 in Figure 3. The simulated mean steady state through level was 17 ng/ml. Following repeated oral administration of a MRF a similar AF was found (AF=2.5 in Figure 4) but higher through levels (32 ng/ml) and much lower fluctuations of DRSP serum levels were found (Fig.4). Higher through levels after repeated administration of the MRF let suggest a decrease of necessary daily doses by about 30 %.

This invention provides a method for combination hormone replacement therapy by administering a solid oral modified release formulation containing 8-PN and DRSP to the patient as a single daily dose.

Still another aspect, this invention provides a method of maintaining therapeutically effective levels of 8-PN and DRSP in human plasma by administering an 8-PN and DRSP containing oral modified release formulation once daily.

The method includes administering an oral modified release formulation including from about 5 to about 80 % by weight 8-PN and less than 5 % by weight DRSP in one dosage form per dose to women, to maintain 8-PN plasma levels from about 0.5 to about 5 ng/ml and to maintain DRSP plasma levels from about 10 to about 60 ng/ml for at least 24 hours wherein the dose is administered once a day.

The formulations of this invention may be either in the form of single unit dosages, e.g. tablets, or in the form of multiple unit dosages, e.g. granulates, pellets or mini-tablets. These multiple unit dosages may be filled in gelatine capsules or compressed to tablets.

The single unit dosages may be produced by powder blending and direct compression into tablets or by powder blending, granulation and compression into tablets. The tablets may be coated by a film.

Multiple unit dosage may be produced by extrusion/spheronization, by layering technique, by rotor granulation, by powder blending and direct compression into mini tablets, by powder blending, granulation and compression into mini tablets, by powder blending, direct compression into mini tablets and film coating or by powder blending, granulation, compression into mini tablets and film coating.

8-Prenylnaringenin can be produced by the method described in WO 2005/037816. Drospirenone can be produced by the method described in US 6,933,395.

### Description of the figures

Fig. 1 shows drug serum levels following a single oral dose of 50 mg 8-Prenylnaringenin in two postmenopausal women; 8-Prenylnaringenin was administered as a 1:1 mixture with lactose (gelatine capsule) at fasted state in the morning, lunch was served 6 hours later. Re-increases in drug serum levels show re-absorption of dose parts collected in the bile fluid and subsequently secreted triggered by lunch.
Fig. 2 shows DRSP serum levels after oral administration of 3 mg DRSP combined with 0.03 mg EE in 13 women. The COC was taken for 13 cycles at a 21 days/7 days regimen. Samples were drawn on day 1 of cycle 1 and on day 21 of cycles 1, 6, 9, and 13. The figure was taken from Blode H, Wuttke W, Loock W, Heithecker R (2000): The European Journal of Contraception and Reproductive Care, 5; 256-264
Fig 3 shows mean (N=13) DRSP serum levels following a single oral administration of 3 mg as a rapidly drug releasing film coated tablet and a simulation of daily repteated administrations; single dose data were generated from day 21, cycle 1 (Blode et al, 2000) by subtraction of prevalue or figures resulting from a prevalue decrease with a half-life of 30 hours. Simulation was performed using the software TopFit 2.0.
Fig. 4 shows the DRSP serum levels following a single oral dose of 3 mg as modified release formulation and a simulation of daily repeated administrations of a MRF; single dose data were generated by a simulation of a zero order release of drug during the first 10 hours after ingestion and a drug level decrease with a half-life of about 30 hours thereafter. Basic PK data for simulation were taken from: Blode H, Wuttke W, Loock W, Heithecker R (2000): The European Journal of Contraception and Reproductive Care, 5; 256-264

### Examples:

### Example 1

Preparation of a modified release formulation containing 8-Prenylnaringenin and Drospirenone

### Production of Mini-Matrix Tablets by Means of Direct Tableting

2.333 mg of 8-Prenylnaringenin
0.046 mg Drospirenone
1.000 mg of Kollidon SR^{®}
1.946 mg of lactose
1.500 mg microcrystalline cellulose
0.070 mg of highly dispersed silicon dioxide
0.105 mg of magnesium stearate

8-Prenylnaringenin, DRSP, Kollidon SR^{®}, lactose and microcrystalline cellulose are sieved individually and mixed in a turbula mixer for 10 minutes. Highly dispersed silicon dioxide, sieved, is added, and all components are mixed in the turbula for another 5 minutes. Magnesium stearate, sieved, is spread on, and all components are mixed in the turbula for another 30 seconds. Tableting of the powder mixture into mini-matrix tablets is carried out by means of an eccentric tablet press or a rotary tablet press.
The release from these mini-tablets is measured by means of the method that is mentioned in Example 4.

### Example 2

### Production of Mini-Matrix Tablets by Means of Direct Tableting

2.333 mg of 8-Prenylnaringenin
0.046 mg Drospirenone
1.000 mg of Kollidon SR^{®}
1.169 mg of magnesium oxide
0.777 mg of lactose
1.500 mg microcrystalline cellulose
0.070 mg of highly dispersed silicon dioxide
0.105 mg of magnesium stearate

8-Prenylnaringenin, DRSP, Kollidon SR^{®}, magnesium oxide, lactose and microcrystalline cellulose are sieved individually and mixed in a turbula mixer for 10 minutes. Highly dispersed silicon dioxide, sieved, is added, and all components are mixed in the turbula for another 5 minutes. Magnesium stearate, sieved, is spread on, and all components are mixed in the turbula for another 30 seconds. Tableting of the powder mixture into mini-matrix tablets is carried out by means of an eccentric tablet press or a rotary tablet press.
The release from these mini-tablets is measured by means of the method that is mentioned in Example 4.

### Example 3

### Production of Mini-Matrix Tablets by Means of Direct Tableting

2.333 mg of 8-Prenylnaringenin
0.046 mg Drospirenone
1.000 mg of Kollidon SR^{®}
1.169 mg of magnesium hydroxide
0.777 mg of lactose
1.500 mg microcrystalline cellulose
0.070 mg of highly dispersed silicon dioxide
0.105 mg of magnesium stearate

8-Prenylnaringenin, DRSP, Kollidon SR^{®}, magnesium hydroxide, lactose and microcrystalline cellulose are sieved individually and mixed in a turbula mixer for 10 minutes. Highly dispersed silicon dioxide, sieved, is added, and all components are mixed in the turbula for another 5 minutes. Magnesium stearate, sieved, is spread on, and all components are mixed in the turbula for another 30 seconds. Tableting of the powder mixture into mini-matrix tablets is carried out by means of an eccentric tablet press or a rotary tablet press.
The release from these mini-tablets is measured by means of the method that is mentioned in Example 4.

### Example 4

### Measurement of the Release of 8-PN and DRSP

Measurement of the active ingredients release from mini-matrix tablets is carried out according to a one-compartment method (basket apparatus), as described in U.S. Pharmacopeia USP XXV. The release of 8-PN and DRSP is examined in phosphate buffer solution, pH 6.8 (composition, see USP XXV). Ten percent (w/w) hydroxypropyl-β-cyclodextrine is added in order to achieve sink conditions and primarily control the drug release by the dosage form.

## Claims

1. Oral modified release formulation containing 8-Prenylnaringenin, Drospirenone, a polymeric matrix, a buffer substance and one or more excipients.

2. Oral modified release formulation containing 8-Prenylnaringenin, Drospirenone a polymeric matrix, a buffer substance and one or more excipients and where the particle size of the compounds is in the range of 0.1 - 750 µm.

3. Oral modified release formulation containing 8-Prenylnaringenin and Drospirenone according to claim 1 or 2 wherein the buffer substance is an alkaline substance.

4. Oral modified release formulation containing 8-Prenylnaringenin and Drospirenone according to claims 1, 2 or 3 wherein said formulation is coated with a polymeric coat that affects the dissolution of active ingredients.

5. Oral modified release formulation according to claims 1 - 4 wherein the polymer matrix is chosen from the group of the following materials: cellulose derivatives, acrylic derivatives, vinyl polymers, polyacrylates, polycarbonates, polyethers, polystyrenes polyanhydrides, polyesters, polyorthoesters, polysaccharides and natural polymers.

6. Oral modified release formulation according to claims 1 - 4 wherein the polymer matrix is chosen from water soluble polyvinylpyrrolidone and water insoluble polyvinylacetate.

7. Oral modified release formulation according to claims 1 - 6 wherein the buffer substance is magnesium oxide, magnesium hydroxide, dihydroxyaluminum aminoacetate, magnesium carbonate, calcium carbonate, sodium ascorbate, magnesium trisilicate, dihydroxyaluminum sodium carbonate, aluminum hydroxide, sodium citrate, potassium phosphate, sodium bicarbonate, disodium hydrogenphosphate or some combinations thereof.

8. Oral modified release formulation according to claims 1 - 7 wherein the formulation contains an additional lubricant.

9. Oral modified release formulation according to claims 1 - 8 wherein the excipient is lactose, calcium phosphate, manitol or starch.

10. Oral modified release formulation according to claims 1 - 9 wherein the formulation contains microcrystalline cellulose as an additional excipient.

11. Oral modified release formulation according to claims 1 - 10 wherein the formulation contains silicon dioxide as flow promoter.

12. Oral modified release formulation according to claims 1 - 11 wherein the particle size of the powder mixtures is between 20 - 400 µm.

13. Use of the oral modified release formulation according to claims 1-12 for the production of a medicament for the combination hormonal contraception where the preferred daytime of ingestion is the evening or bedtime.

14. Use of the oral modified release formulation according to claims 1-13 for the production of a medicament for the treatment of symptoms of estrogen deficiency

15. Use of the oral modified release formulation according to claims 1-13 for the production of a medicament for the treatment of menopausal symptoms

16. Use of the oral modified release formulation according to claims 1-13 for the production of a medicament for the treatment of hot flushes

17. Use of the oral modified release formulation according to claims 1-16 for the production of a medicament for the combination hormone replacement therapy where the active treatment phase is between 21 and 25 days.

18. Use of the oral modified release formulation according to claims 1-16 for the production of a medicament for the combination hormone replacement therapy where the active treatment phase is continuous.

19. Use of the oral modified release formulation according to claims 1-18 where 8-PN is replaced by any derivative of 8-Prenylnaringenin.

20. Use of the oral modified release formulation according to claims 1-18 where Drospirenone is replaced by any any derivative of Drospirenone.
